(19) Europäisches Patentamt
European Patent Office
Office européen des brevets

(11) **EP 3 678 551 B1**

(12) **EUROPEAN PATENT SPECIFICATION**

(45) Date of publication and mention
of the grant of the patent:
**28.06.2023 Bulletin 2023/26**

(21) Application number: **18853978.7**

(22) Date of filing: **22.08.2018**

(51) International Patent Classification (IPC):
**A61B 7/04** (2006.01)

(52) Cooperative Patent Classification (CPC):
**H04R 1/46; A61B 7/04**

(86) International application number:
**PCT/IB2018/056335**

(87) International publication number:
**WO 2019/048960 (14.03.2019 Gazette 2019/11)**

(54) **ELECTRONIC STETHOSCOPE WITH ENHANCED FEATURES**

ELEKTRONISCHES STETHOSKOP MIT VERBESSERTEN FUNKTIONEN

STÉTHOSCOPE ÉLECTRONIQUE À CARACTÉRISTIQUES AMÉLIORÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.09.2017 US 201762554041 P
16.04.2018 US 201815953502**

(43) Date of publication of application:
**15.07.2020 Bulletin 2020/29**

(73) Proprietor: **Sanolla Ltd
3688519 Nesher (IL)**

(72) Inventors:
• **ADLER, Doron**
**Haifa (IL)**
• **KAGAN, Igor**
**Kiriat Bialik (IL)**
• **SALOMON, Ezra**
**Rakefet (IL)**
• **ADLER, Omri**
**Haifa (IL)**
• **LINHARD, David**
**Haifa (IL)**

(74) Representative: **Beck Greener LLP
Fulwood House
12 Fulwood Place
London WC1V 6HR (GB)**

(56) References cited:
**WO-A1-2017/141165       WO-A1-2017/141165
WO-A2-02/09586          CN-A- 107 510 473
KR-A- 20120 040 530     US-A1- 2001 030 077
US-A1- 2002 071 570     US-A1- 2003 002 685
US-A1- 2007 058 818     US-A1- 2012 209 132
US-A1- 2018 228 468     US-B2- 8 920 343**

## Description

## FIELD OF THE INVENTION

The present invention relates generally to methods, apparatus and software for medical diagnosis, and particularly to electronic stethoscopes and methods for their use.

## BACKGROUND

Auscultation has been a key technique in medical diagnosis for centuries. In auscultation, the medical practitioner listens to the internal sounds of the body, typically using a stethoscope. Auscultation is most commonly performed for the purpose of examining the circulatory and respiratory systems, and thus diagnosing conditions of the heart and lungs in particular. In more recent years, electronic stethoscopes and methods of digital processing of body sounds have become available, in order to enhance and supplement the practitioner's auditory capabilities.

For example, U.S. Patent 5,853,005 describes an acoustic monitoring system in which a transducer in communication with fluid in a pad is held in close contact against a sound or movement source and monitors acoustic signals transferred into the fluid. The signal pattern is monitored aurally and/or compared to predetermined reference patterns.

As another example, U.S. Patent 6,699,204 describes a device for analyzing auscultation sounds, in particular respiratory sounds. The device comprises an input receiving a sound signal sampled in intensity levels each associated with a selected time, and storage means comprising a processing module for evaluating, in cooperation with computing means, a set of transformed intensity levels, each associated with a predetermined sound frequency. It further comprises an output connected to the storage means for delivering each transformed intensity level in correspondence with an associated frequency, and means for representing intensity levels transformed on the basis of frequencies, to obtain a spectral representation of the auscultation sound.

U.S. Patent Application Publication 2005/0222515 describes methods of analyzing patient's heart, using a cardiovascular sound signature in diagnosis at the early stages of cardiac dysfunctions. The invention is said to present cardiovascular sounds in time and frequency while keeping signal resolution equally strong in both directions.

PCT International Publication WO 2017/141165 describes apparatus for detecting sound waves emanating from a body of a subject. The apparatus includes a housing and a membrane, disposed at an opening of the housing. The membrane is configured to deflect, when an outer face of the membrane contacts the body, responsively to the sound waves impinging on the membrane. The apparatus further includes a piezoelectric microphone, disposed within the housing, configured to detect vibrations of air caused by the deflection of the membrane, and to generate a microphone output in response thereto. An accelerometer, disposed on an inner face of the membrane, deflects, along with the membrane, at frequencies below a minimum frequency that is detectable by the piezoelectric microphone, and generate an accelerometer output in response thereto. A processor processes the microphone output and the accelerometer output, and generates, responsively to the processing, a sound signal that represents the impinging sound waves.

US2003/002685 discloses an electronic auscultation aid that improves the performance and convenience of auscultation. The auscultation aid may include means for emphasizing sounds that may be associated with pathological or pre-pathological conditions. The auscultation aid may include a wireless interface for distributing a detected signal to one or more remote devices. The auscultation aid may comprise a mount for temporary attachment to a conventional acoustic stethoscope. The auscultation aid may include an apparatus for switching between active and sleep modes for the conservation of battery life. US2003/002685 discloses a device and method according to the preambles of independent claims 1 and 13 respectively.

US2007/058818 discloses a stethoscope apparatus including an integrally mounted speaker and microphone. The speaker can reproduce sounds obtained by the microphone.

## SUMMARY

The invention is as defined in the claims. Embodiments of the present invention that are described hereinbelow provide apparatus and methods for collecting, processing and analyzing signals from the body surface.

There is therefore provided, in accordance with an embodiment of the invention, a medical device, which includes a case, having a front surface that is configured to be brought into contact with a body of the living subject. A microphone is contained in the case and configured to sense acoustic waves emitted from the body and to output an acoustic signal in response thereto. A proximity sensor is configured to output a proximity signal indicative of contact between the front surface and the body. At least one speaker is configured to output audible sounds. Processing circuitry is coupled to detect, in response to the proximity signal, that the front surface is in contact with the body, and in response to the detected contact, to process the acoustic signal so as to generate an audio output and to convey the audio output to the at least one speaker.

In some embodiments, the device includes a motion sensor, which is contained in the case and is configured to output a motion signal in response to motion of the front surface. The processing circuitry can be configured to process the motion signal in order to detect a respiratory cycle of the subject. In a disclosed embodiment, the front surface includes a membrane, which is configured to vibrate in response to the acoustic waves emitted from the body, and the motion sensor is configured to sense the motion of the membrane, and the processing circuitry is configured to process the motion signal in order to detect an infrasonic component of the acoustic waves.

In one embodiment, the processing circuitry is configured to process the acoustic signal so as to generate a frequency-stretched audio output in which infrasonic frequency components of the acoustic signal are shifted to audible frequencies while preserving the periodicity of the periodic physiological activity, and to convey the frequency-stretched audio output to the at least one speaker.

Additionally or alternatively, the processing circuitry is configured to power down one or more components of the device when the proximity signal indicates that the front surface is not in contact with the body, and to power up the one or more components when the proximity signal indicates that contact has been made between the front surface and the body.

In some embodiments, the device includes a pair of earphones, which are configured to be inserted in respective ears of an operator of the device and to convey the audible sounds to the ears. In one such embodiment, the processing circuitry is configured to detect that the earphones have been spread apart and to power up one or more components of the device in response thereto.

Additionally or alternatively, the processing circuitry is configured to detect, in response to the proximity signal, a quality of the contact between the front surface and the body, and to output an indication of the detected quality to an operator of the device.

According to the invention as defined in claim 1, the proximity sensor includes at least one emitter, which is configured to emit optical radiation, including infrared radiation, toward the body, and a detector, which is configured to sense the optical radiation reflected from the body. The processing circuitry is configured to measure a level of oxygen saturation in blood of the subject responsively to the reflected radiation sensed by the detector.

Typically, the case has a rear surface opposite the front surface, and the device includes a touch-sensitive display screen mounted on the rear surface of the case. In some embodiments, the processing circuitry is configured to present a user interface on the display screen and to receive an input from an operator of the device via the display screen indicating a processing mode and frequency shift to be applied in processing of the acoustic signal. The processing mode can be selected from a group of processing modes consisting of a cardiac signal processing mode and a respiratory signal processing mode.

Additionally, the processing circuitry is configured to compute, responsively to the acoustic signal, and render to the display screen an acoustic signature, including a graphical representation of a spectral distribution of an energy of the acoustic waves over a period of physiological activity. In one embodiment, the processing circuitry is configured to compute respective autocorrelations of the acoustic signal for a plurality of different times within a period of the physiological activity, to transform the respective autocorrelations to a frequency domain, and to render the acoustic signature to the display screen responsively to the transformed autocorrelations. In some embodiments, the acoustic waves include an infrasonic component, and the spectral distribution includes the energy at infrasonic frequencies. In a disclosed embodiment, the graphical representation includes a plot having a frequency axis and a time axis defining a time-frequency plane and presenting a value of the energy at each point in the time-frequency plane.

There is also provided, in accordance with an embodiment of the invention as defined in independent claim 13, a method for collecting, processing and analyzing signals from a body surface, which includes receiving, from a microphone in a case having a front surface that is brought into contact with a body of a living subject, an acoustic signal in response to acoustic waves emitted from the body. A proximity signal is indicative of contact between the front surface of the case and the body. In response to the proximity signal, contact of the front surface with the body is detected. In response to the detected contact, the acoustic signal is processed so as to generate and convey an audio output to at least one speaker; wherein receiving the proximity signal comprises emitting optical radiation toward the body, and sensing the optical radiation reflected from the body; and wherein emitting the optical radiation comprises emitting infrared radiation;characterised in that the method comprises measuring a level of oxygen saturation in the blood of the subject using the sensed reflected radiation.

The present invention will be more fully understood from the following detailed description of the embodiments thereof, taken together with the drawings in which:

## BRIEF DESCRIPTION OF THE DRAWINGS

Fig. 1 is a schematic pictorial illustration showing an electronic stethoscope in clinical use, in accordance with an embodiment of the invention;
Fig. 2 is a block diagram that schematically shows elements of an electronic stethoscope, in accordance with an embodiment of the invention;
Fig. 3 is a schematic pictorial illustration showing details of the head of an electronic stethoscope, in accordance with an embodiment of the invention; and
Figs. 4 and 5 are schematic pictorial illustrations showing operation of an interactive display on the head of an electronic stethoscope, in accordance with an embodiment of the invention.

## DETAILED DESCRIPTION OF EMBODIMENTS

OVERVIEW

The stethoscope is generally the first tool that the medical practitioner deploys in attempting to diagnose conditions of the circulatory and respiratory systems. Stethoscopes that are known in the art, however, including electronic stethoscopes, are limited by the user's auditory capabilities and diagnostic skills. Signals available at the body surface due to physiological activity contain a wealth of diagnostic information that is not exploited either by traditional auscultation or by electronic stethoscopes that are known in the art.

Embodiments of the present invention that are described herein provide medical devices that offer functionalities of electronic stethoscopes with enhanced diagnostic capabilities and ease of use. The devices described below include a case, whose front surface is brought into contact with the body of a living subject. The case contains a microphone, which senses acoustic waves emitted from the body, as well as a proximity sensor, which outputs a proximity signal indicative of contact between the front surface of the case and the body. At least one speaker, in or connected to the case, outputs audible sounds. Typically (although not necessarily), the audible sounds are conveyed to earphones, which are inserted in the ears of an operator, for example by acoustic tubes connecting the speaker to the earphones or by integrating a pair of speakers into the earphones.

Based on the proximity signal, processing circuitry, which may be contained in the case, detects, that the front surface of the case is in contact with the body. In response to the detected contact, the circuitry processes acoustic signals output by the microphone so as to generate an audio output, which is conveyed to the speaker or speakers. This sort of contact sensing is useful in ensuring good-quality contact between the front surface of the case and the body. The processing circuitry outputs an indication of the contact quality that is detected in this manner to an operator of the device.

Additionally, the processing circuitry can make use of the proximity signal in reducing power consumption, by powering down one or more components of the device when the proximity signal indicates that the front surface is not in contact with the body, and powering them up only when the proximity signal indicates that contact has been made between the front surface of the case and the body. Further additionally or alternatively, for purposes of power saving, the processing circuitry may detect that the earphones of the stethoscope have been spread apart - indicating that a practitioner is preparing to use the device - and may power up components of the device in response thereto. These measures are useful not only in conserving power, but also in relieving the practitioner of the need to continually switch the device on and off.

According to the invention, the proximity sensor comprises one or more emitters, which emit optical radiation toward the body, and a detector, which senses the optical radiation reflected from the body and outputs the proximity signal on this basis. The emitter or emitters output appropriate wavelengths, including in the near-infrared range, therefore the processing circuitry can use the output of the detector to measure the level of oxygen saturation in the subject's blood, so that the device functions as a pulse oximeter, in addition to it audio functionality.

In the embodiments that are described below, the head of the stethoscope also contains a motion sensor, which outputs a motion signal in response to motion of the front surface of the case. The processing circuitry may process this motion signal in order to detect respiratory motion, and thus extract the respiratory cycle of the subject. Additionally or alternatively, the front surface of the case may comprise a membrane, which vibrates in response to the acoustic waves emitted from the body. The motion sensor senses this vibratory motion, and the processing circuitry can then process the motion signal in order to detect an infrasonic component of the acoustic waves emitted from the body.

Further additionally, the processing circuitry may process acoustic signals from the microphone and/or the motion sensor so as to generate a frequency-stretched audio output, in which infrasonic frequency components of the acoustic signal are shifted to audible frequencies while preserving the periodicity of the periodic physiological activity. This frequency-stretched audio output is conveyed to the speaker or speakers.

In some embodiments, the device comprises a touch-sensitive display screen on the rear surface of the case. The processing circuitry can present a user interface on the display screen and receive inputs from the operator of the device via the display screen. These inputs may indicate, for example, a processing mode, such as a cardiac or respiratory signal processing mode, and/or a frequency shift to be applied in processing of the acoustic signals from the microphone. Additionally or alternatively, the processing circuitry may compute, based on the acoustic signals, an acoustic signature, and may render this acoustic signature to the display screen. The acoustic signature typically comprises a graphical representation of the spectral distribution of the energy of the acoustic waves over a period of physiological activity, such as a heart cycle or respiration cycle.

In the embodiments that are shown in the figures and described below, the features described above, as well as other inventive aspects of the present invention, are shown in combination, by way of example, in the context of a particular design of an electronic stethoscope. Although this combination of features in a single device is advantageous, in terms of the range of capabilities and benefits offered to the operator of the device, those skilled in the art will understand that subsets of these features, including individual features among those described above, may be incorporated in an electronic stethoscope or other medical device, independently of the remaining features. All such individual features and combinations thereof are considered to be within the scope of the present invention, even if not enumerated specifically in the present description.

SYSTEM DESCRIPTION

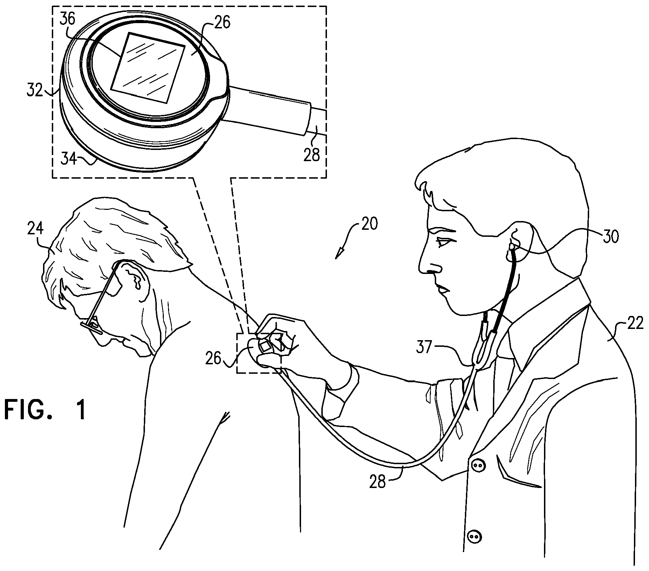

Fig. 1 is a schematic pictorial illustration showing the use of a digital stethoscope 20, in accordance with an embodiment of the invention. A practitioner 22 brings a head 26 of stethoscope 20 into contact with the body of a patient 24. Processing circuitry in head 26 outputs an audio signal via a cable 28 extending from head 26 to one or more speakers, which are typically integrated in or acoustically coupled to earphones 30. The two earphones 30 are joined at a springloaded junction 37, and thus open apart and fit into the practitioner's ears as would the earphones of a conventional stethoscope.

As shown in the inset, head 26 comprises a case 32, which contains an acoustic transducer, such as a suitable microphone 34, which contacts the body of patient 24 and thus senses acoustic waves emitted from the body. Microphone 34 may be of any suitable type that is known in the art, for example a piezoelectric sensor or a MEMS (micro-electro-mechanical systems) microphone, which is sensitive not only to audible frequencies, but also to infrasonic frequencies going down to about 5 Hz. On the other side of head 26, a user interface, such as a touch-sensitive display 36, enables practitioner 22 to control the functions of stethoscope 20 and displays data, such as acoustic signatures of heart and/or breath sounds sensed by microphone 34. Other components and functions of head 26 are described with reference to the figures that follow.

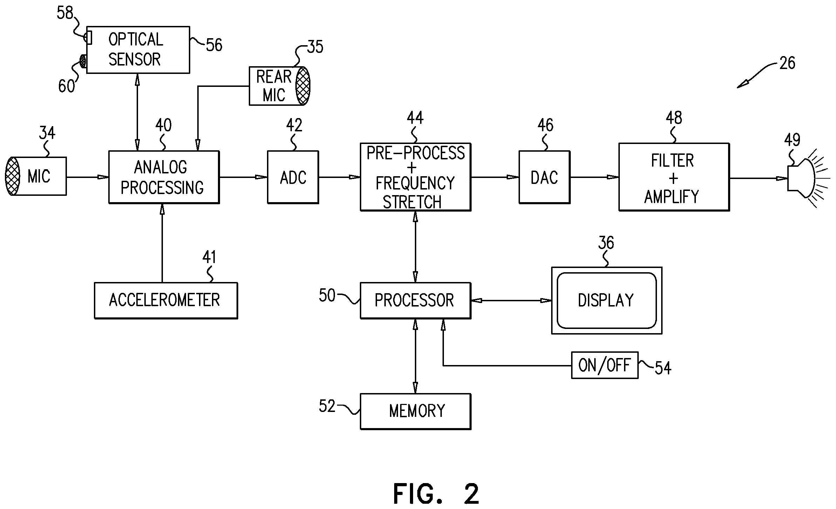

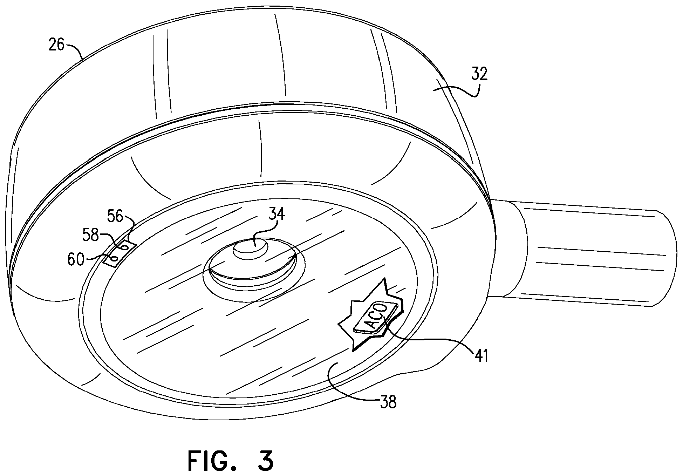

Reference is now made to Figs. 2 and 3, which schematically illustrate functional elements of stethoscope 20, in accordance with an embodiment of the invention. Fig. 2 is a block diagram, while Fig. 3 is a pictorial detail view of head 26 of stethoscope 20. In the present embodiment, the elements shown in Fig. 2 are assumed to be contained inside head 26, within case 32, with the possible exception of one or more speakers 49 (and possibly an on/off switch 54, as explained below). Alternatively, however, certain components may be housed in other parts of stethoscope 20 or in an external processing unit.

The front surface of head 26, which is brought into contact with the patient's body, comprises a membrane 38. Microphone 34 is typically mounted at the center of this membrane. Acoustic waves from the body impinge on membrane 38, causing the membrane to deflect. The deflection of membrane 38 is translated into an acoustic signal, which is processed, as described in detail below, to generate an audio output, which is played via speaker 49 to earphones 30 in the example shown in Fig. 1. Features of membrane 38 and microphone 34 are described further in the above-mentioned PCT International Publication WO 2017/141165.

An analog front end 40 performs analog processing functions, including filtering, buffering and amplification of the electrical signals output by microphone 34. Optionally, head 26 also contains a rear microphone 35, in a location that is physically separated from membrane 38, which captures background sounds. These background sounds are subtracted from the signals from microphone 34. The subtraction may be carried out either in analog front end 40 or digitally, for example by adaptive filtering in either the time or frequency domain, following digitization of the signals, as described below.

Head 26 also comprises an inertial sensor, such as an integrated circuit accelerometer 41, which measures motion of head 26 and low-frequency vibrations of membrane 38. Analog front end 40 processes the signals output by the inertial sensor, as well. Accelerometer 41 is typically disposed on the inner side of membrane 38, and may serve at least two functions: both detecting movement of the chest caused by respiration, which causes head 26 to move cyclically at the respiration frequency, and detecting deflections of membrane 38 at vibrational frequencies below the minimum frequency that is detectable by microphone 34. The accelerometer and microphone thus complement one another, in that the accelerometer detects sound at very low frequencies that are not detectable by the microphone, and the microphone detects sound at higher frequencies that are not detectable by the accelerometer.

An analog/digital converter (ADC) 42 digitizes the acoustic and inertial signals, and possibly also other analog inputs. For purposes of audio enhancement and analysis, a digital preprocessing circuit 44 transforms the digitized signals to the frequency domain, for example by computing a short-time Fourier transform (STFT) over successive time windows of the signals. In addition, circuit 44 can perform digital filtering functions, such as noise suppression, and "frequency stretching": shifting infrasonic frequency components to the audible frequency range, as described further hereinbelow. Following these filtering and frequency stretching steps, circuit 44 converts the frequency-domain samples back to the time domain. For these purposes, circuit 44 typically comprises digital processing logic, which may be hard-wired or programmable; but alternatively, at least some of the functions of circuit 44 may be carried out on a programmable processor under the control of software or firmware.

A digital/analog converter (DAC) 46 converts the stream of processed time-domain samples to analog form. In this manner, practitioner can choose to hear audible versions of the infrasonic frequency components captured by microphone 34 and accelerometer 41, following frequency-stretching to the audible range, in addition to or instead of the audible frequency components themselves that are captured by the microphone. An analog output circuit 48 filters and amplifies the analog signal to generate an electrical audio output to speaker or speakers 49.

A programmable processor 50 receives the stream of samples - in either the time domain or the frequency domain, or both - from digital preprocessing circuit 44. Processor 50 is coupled to a memory 52, which typically comprises non-volatile memory, such as flash memory, containing software or firmware to control the operations of processor 50. In addition, memory 52 typically comprises volatile random-access memory (RAM), which is used by processor 50 to store the digital samples received from circuit 44, as well as processing results.

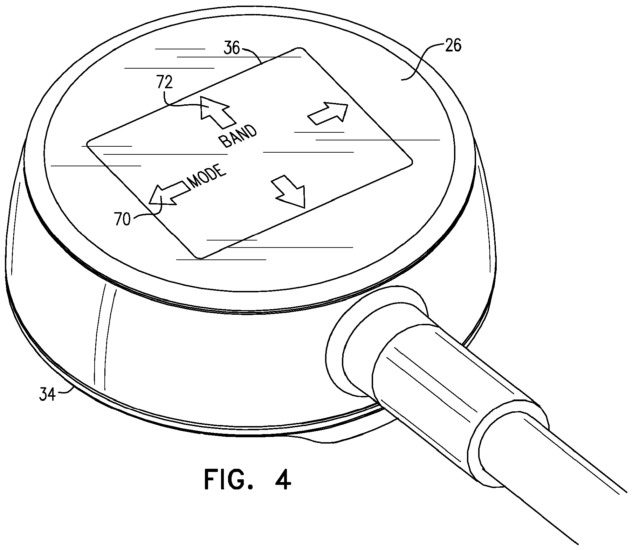

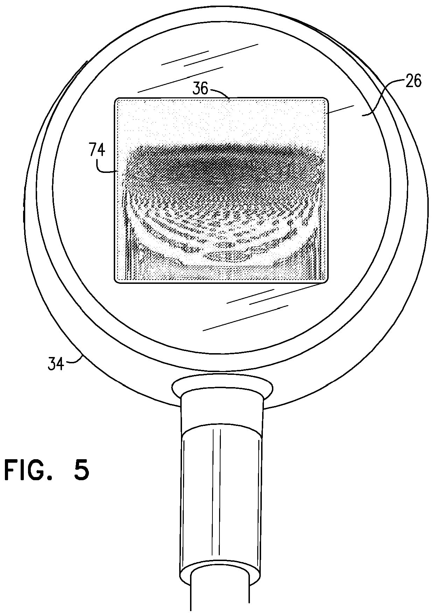

Based on the digitized samples, processor 50 can compute acoustic signatures, representing the spectral distribution of the energy of the acoustic waves over the period of the cardiac or respiratory cycle, as described further hereinbelow. Processor 50 renders a graphical representation of the acoustic signature to display 36 and/or outputs the signature information via a communication link (not shown). In addition, processor 50 may receive and carry out user instructions, for example in response to finger gestures on the touch screen of display 36. These functions of display 36 are illustrated and described further hereinbelow with reference to Figs. 4 and 5.

The processing components shown Fig. 2, including analog front end 40, ADC 42, digital preprocessing circuit 44, DAC 46, analog output circuit 48, processor 50 and memory 52, are collectively and individually referred to herein as "processing circuitry." These components are typically implemented in integrated circuits, as are known in the art, which are mounted together on a printed circuit board within case 32. Alternatively, other implementations of these functional components will be apparent to those skilled in the art after reading the present description and are considered to be within the scope of the present invention. Although Fig. 2 shows a certain arrangement of functional blocks for the sake of conceptual clarity, the functions of at least some of these blocks may be combined into a single integrated circuit chip or, alternatively, split among multiple chips.

Typically (although not necessarily), the functions of stethoscope 20, and specifically of the processing circuitry described above, are powered by a battery (not shown). In order to conserve battery power, it is desirable that at least some of the components of the processing circuitry be powered down automatically when not in use, and then powered up automatically when needed, without requiring practitioner 22 to operate an on/off switch. For this purpose, stethoscope 20 comprises an on/off sensor 54, which detects an action made by practitioner in preparation for applying head 26 to patient 24. For example, sensor 54 may comprise an electro-mechanical component, which is connected to junction 37 (Fig. 1) and outputs a signal when earphones 30 are spread apart. In response to this signal, processor 50 powers up the processing circuitry. When the earphones are released, the processing circuitry may be powered down.

Additionally or alternatively, head 26 comprises a proximity sensor 56, which outputs a proximity signal indicative of contact between the front surface of case 32 and the patient's body.

The proximity sensor 56 is an optical sensor, comprising one or more emitters 58, which emit optical radiation toward the body, and a detector 60, which senses the optical radiation reflected from the body. Detector 60 thus outputs a signal (referred to herein as a "proximity signal") that is indicative of the proximity of sensor 56 to the patient's skin. Alternatively or additionally, head 26 may comprise other types of proximity sensors, such as a strain gauge or other pressure sensor, which measures the pressure of head 26 against the patient's body.

Based on the signal from detector 60, processor 50 is able to determine whether or not head 26 is in contact with the patient's body. Thus, stethoscope 20 may automatically convey the processed audio output to speaker 49 (and from there to earphones 30) only when the front surface of head 26 is in proper contact with the body. Processor 50 may otherwise power down certain components of the stethoscope when the signal from detector 60 indicates that the front surface of head 26 is not in contact with the body, and may then power up the components when contact is made. This functionality may be instead of or in addition to that of on/off sensor 54.

As another option, processor 50 may assess, based on intensity and/or other features of the reflected light sensed by detector 60, the quality of the contact between the front surface of head 26 and the body. Processor 50 may thus be able to measure whether practitioner 22 is pressing head 26 against the patient's body with sufficient force, or perhaps too much force, and to output an indication of the detected quality to practitioner 22. For example, processor 50 may render a graphical and/or alphanumeric output to display 36, indicating, for example, that the contact between head 26 and the patient's skin is too weak, or possibly too strong.

According to the invention, processor 50 processes the signals output by detector 60 in order to measure the level of oxygen saturation in the blood of patient 24. For this purpose, emitter 58 typically emits optical radiation at appropriate wavelengths, such as an infrared wavelength and a visible wavelength, for example at 940 nm and 660 nm, as is known in the art. Emitter 58 may comprise a pair of suitable light-emitting diodes for this purpose. Emitter 58 emits the two wavelengths in sequential alternation, and processor 50 compares the signals output by detector 60 in response to the two wavelengths in order to calculate the approximate oxygen saturation level. Processor 50 outputs an indication of the saturation level to display 36, along with other data outputs and controls.

MODES OF OPERATION

Fig. 4 is a schematic pictorial illustration of stethoscope head 26, showing elements of a user interface presented by processor 50 on touch-sensitive display 36, in accordance with an embodiment of the invention. The user interface includes a mode selection control 70 and a band selection control 72, which are operated by finger gestures made by practitioner 22 against the display screen. For example, the practitioner may swipe his finger left or right on the screen to change the processing mode, and may swipe his finger up or down to choose a frequency shift to be applied in processing of the acoustic signals captured by head 26. The processing mode can be selected for example, to be a cardiac signal processing mode or a respiratory signal processing mode. Band selection control 72 allows the user to turn frequency stretching on and off, in order to shift infrasonic signals to the audible range, as well as to choose the degree of stretching to be applied. In addition, display 36 can present other controls and data outputs, as will be apparent to those skilled in the art after reading the present description.

As explained above (in reference to Fig. 2), analog front end 40 receives the electrical signals output by microphones 34 and 35 and by accelerometer 41, and ADC 42 digitizes the signals. The time-domain digital samples are input to digital preprocessing circuit 44, which performs preprocessing functions that may include subtracting background audio signals captured by microphone 35. Circuit 44 then transforms the digitized signals to the frequency domain, using a short-time Fourier transform (STFT), for example.

To extend the spectrum down to the low infrasonic range, the vibrational signals captured by accelerometer 41 may similarly be transformed and incorporated into the spectrum together with the (background-subtracted) signals from microphone 34. In processing the accelerometer output, circuit 44 typically applies appropriate frequency filters to this output, such as to differentiate chest movements (which typically have a frequency of 0.1-0.2 Hz) from deflections of membrane 38 having higher or lower frequencies. The membrane-deflection portion of the accelerometer output can then be integrated with the output from microphone 34. Processor 50 can analyze the chest-movement portion of the accelerometer output, such as to identify the various stages in the respiratory cycle of the subject.

Formally, the STFT F(m,k) of the time-domain audio signal f[n], is computed by applying the formula:

$$F(m,k) = \sum_n f[n] * \mathrm{w}[n-m] * \mathrm{e}^{-jkn}$$

Here m and k are both discrete variables (m being the time variable and k being the frequency variable), and w[n] is a suitable window function, such as a Hamming, Gaussian, or Hann window function.

To stretch the infrasonic signals into the audible range, circuit 44 interpolates the frequency spectrum F(m,k) in the frequency domain by a factor R in order to yield interpolated spectrum $F_r(m,k)$, in which the infrasonic components are shifted to audible frequencies. Generally speaking, R may take any rational value, but typical values of R are in the range between 2 and 20, in order to shift infrasonic frequency components to the low end of the audible range. Assuming that the infrasonic frequency components sensed by microphone 34 have frequencies extending at least down to 5 Hz, and circuit 44 will use a value of R such that the infrasonic frequency component at 5 Hz will be shifted to a frequency of at least 20 Hz.

Different values of R can be used depending upon whether practitioner 22 wishes to hear respiratory or cardiac sounds. For example, respiratory sounds typically fall within the range of 12-2000 Hz, which can be shifted to the audible range of 24-4000 Hz by setting R=2. As another example, heart sounds typically fall within the range of 5-200 Hz, which can be shifted to the audible range of 40-1600 Hz by setting R=8. Optionally, practitioner 22 may select the value of R (for example, toggling between preset values for different types of physiological activity) by entering an appropriate user input to stethoscope 20, using controls 70 and 72 on the touch screen of display 36, for example, as illustrated in Fig. 4.

Stretching the frequency content of this acoustic signal, so as to slow down or accelerate the temporal evolution of a sound without altering its time period, requires an explicit separation of temporal and spectral information. To carry out the required time domain preservation, digital preprocessing circuit 44 first computes $f_r[n]$, the inverse STFT of $F_r(m,k)$, as given by the formula:

$$f_r[n] = \frac{1}{2\pi} \sum_m \sum_k F_r(m,k) * \mathrm{e}^{jkn}$$

Circuit 44 then decimates the sequence of time-domain samples $f_r[n]$ by the same factor R as was used in interpolation of the frequency spectrum to give a frequency-stretched signal $f_s[n]$, which has the same number of samples, and hence the same periodicity, as the original f[n], but is stretched to higher frequencies. (This decimation is equivalent to replaying the frequency-shifted sequence of time-domain samples $f_r[n]$ at R times the original sample rate.) DAC 46 converts $f_s[n]$ to analog form, and analog output circuit 48 amplifies and inputs this signal to speaker or speakers 49.

COMPUTATION AND DISPLAY OF ACOUSTIC SIGNATURES

Fig. 5 is a schematic pictorial illustration of stethoscope head 26, showing an acoustic signature 74 on display 36, in accordance with an embodiment of the invention. Acoustic signature 74 is a graphical representation of the spectral distribution of the energy of the acoustic waves over a period of physiological activity. In the example shown in Fig. 5, signature 74 is derived from chest sounds (including infrasonic components) due to respiratory activity, and is thus indicative of the patient's respiratory patterns. Signature 74, by way of example, is indicative of chronic obstructive pulmonary disease (COPD). Alternatively, practitioner 22 may select the operating mode of stethoscope 20, using mode selection control 70 on the touch screen interface of display 36, for example, to obtain and present an acoustic signature of the patient's cardiac activity.

As explained in detail in the above-mentioned U.S. Patent Application 15/953,502, acoustic signature 74 is based on the autocorrelation spectrum of acoustic signals received from microphone 34 over the period of a physiological activity of interest, such as the autocorrelation of heart sounds over the cardiac cycle or breath sounds over the respiratory cycle. For this purpose, for example, processor 50 can detect the respiratory motion of the patient's thorax using the output of accelerometer 41 and thus extract the period of the patient's respiratory activity. Additionally or alternatively, the period of the activity, based on the digitized signal x(n) from microphone 34 or accelerometer 41, can itself be computed by finding and analyzing significant peaks in the autocorrelation function.

To compute the acoustic signature of the respiratory activity, processor 50 builds a two-dimensional (2D) autocorrelation matrix based on the digitized samples of the electrical signals from microphone 34 and possibly accelerometer 41, and then applies an appropriate transform to the 2D autocorrelation matrix to generate a matrix representation of the spectral (frequency) distribution of the energy of the acoustic waves over the respiratory period. This matrix constitutes the spectral signature of the patient's cardiac or respiratory activity.

Processor 50 can then render a graphical representation of this signature to display 36 or to a separate, external display (not shown). Typically, this graphical representation comprises a plot having a frequency axis and a time axis, which together define a time-frequency plane. The value of the energy is presented in the plot at each point in the time-frequency plane. Signature 74, as shown in Fig. 5, is one example of such a graphical representation, while other examples are shown in the above-mentioned U.S. Patent Application 15/953,502. These acoustic signatures can thus assist the practitioner in making fast, accurate diagnoses and assessing changes in the patient's condition thereafter.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. A medical device (20), comprising:

   a case (32), having a front surface that is configured to be brought into contact with a body of a living subject (24);
   a microphone (34), which is contained in the case and configured to sense acoustic waves emitted from the body and to output an acoustic signal in response thereto;
   a proximity sensor (56), which is configured to output a proximity signal indicative of contact between the front surface and the body;
   at least one speaker (49) configured to output audible sounds; and
   processing circuitry (50), which is coupled to detect, in response to the proximity signal, that the front surface is in contact with the body, and in response to the detected contact, to process the acoustic signal so as to generate an audio output and to convey the audio output to the at least one speaker
   wherein the proximity sensor (56) comprises at least one emitter (58), which is configured to emit optical radiation, including infrared radiation, toward the body, and a detector (60), which is configured to sense the optical radiation reflected from the body,
   **characterised in that** the processing circuitry is also configured to measure a level of oxygen saturation in blood of the subject using the reflected radiation sensed by the detector.

2. The device according to claim 1, and comprising a motion sensor, which is contained in the case (32) and is configured to output a motion signal in response to motion of the front surface.

3. The device according to claim 2, wherein the processing circuitry (50) is configured to process the motion signal in order to detect a respiratory cycle of the subject.

4. The device according to claim 2, wherein the front surface comprises a membrane (38), which is configured to vibrate in response to the acoustic waves emitted from the body, and wherein the motion sensor is configured to sense the motion of the membrane, and the processing circuitry is configured to process the motion signal in order to detect an infrasonic component of the acoustic waves.

**5.** The device according to claim 1, wherein the processing circuitry (50) is configured to process the acoustic signal so as to generate a frequency-stretched audio output in which infrasonic frequency components of the acoustic signal are shifted to audible frequencies while preserving the periodicity of the periodic physiological activity, and to convey the frequency-stretched audio output to the at least one speaker.

**6.** The device according to claim 1, wherein the processing circuitry (50) is configured to power down one or more components of the device when the proximity signal indicates that the front surface is not in contact with the body, and to power up the one or more components when the proximity signal indicates that contact has been made between the front surface and the body.

**7.** The device according to claim 1, and comprising a pair of earphones (30), which are configured to be inserted in respective ears of an operator of the device and to convey the audible sounds to the ears, wherein the processing circuitry is configured to detect that the earphones have been spread apart and to power up one or more components of the device in response thereto.

**8.** The device according to claim 1, wherein the processing circuitry (50) is configured to detect, in response to the proximity signal, a quality of the contact between the front surface and the body, and to output an indication of the detected quality to an operator of the device.

**9.** The device according to any of claims 1-8, wherein the case has a rear surface opposite the front surface, and wherein the device comprises a touch-sensitive display screen (36) mounted on the rear surface of the case.

**10.** The device according to claim 9, wherein the processing circuitry is configured to present a user interface on the display screen (36) and to receive an input from an operator of the device via the display screen (36) indicating a processing mode and frequency shift to be applied in processing of the acoustic signal, wherein the processing mode is selected from a group of processing modes consisting of a cardiac signal processing mode and a respiratory signal processing mode.

**11.** The device according to claim 9, wherein the processing circuitry is configured to compute, responsively to the acoustic signal, and render to the display screen (36) an acoustic signature, comprising a graphical representation of a spectral distribution of an energy of the acoustic waves over a period of physiological activity.

**12.** The device according to claim 11, wherein the acoustic waves comprise an infrasonic component, and wherein the spectral distribution includes the energy at infrasonic frequencies.

**13.** A method for collecting, processing and analyzing signals from a body surface, comprising:

receiving, from a microphone (34) in a case (32) having a front surface that is brought into contact with a body of a living subject (24), an acoustic signal in response to acoustic waves emitted from the body;
receiving a proximity signal indicative of contact between the front surface of the case and the body;
detecting, in response to the proximity signal, that the front surface is in contact with the body; and
in response to the detected contact, processing the acoustic signal so as to generate and convey an audio output to at least one speaker;
wherein receiving the proximity signal comprises emitting optical radiation toward the body, and sensing the optical radiation reflected from the body; and
wherein emitting the optical radiation comprises emitting infrared radiation;
**characterised in that** the method comprises measuring a level of oxygen saturation in the blood of the subject using the sensed reflected radiation.

**Patentansprüche**

**1.** Medizinische Vorrichtung (20), umfassend:

ein Gehäuse (32) mit einer Vorderseitenfläche, die dazu eingerichtet ist, mit einem Körper eines lebenden Subjekts (24) in Kontakt gebracht zu werden;
ein Mikrofon (34), das in dem Gehäuse enthalten und dazu eingerichtet ist, von dem Körper ausgesendete akustische Wellen zu erfassen und in Reaktion darauf ein akustisches Signal auszugeben;
einen Näherungssensor (56), der dazu eingerichtet ist, ein Annäherungssignal auszugeben, das einen Kontakt zwischen der Vorderseitenfläche und dem Körper anzeigt;
mindestens einen Lautsprecher (49), der dazu eingerichtet ist, hörbare Töne auszugeben; und
eine Verarbeitungsschaltung (50), die gekoppelt ist, um als Reaktion auf das Annäherungssignal zu erkennen, dass die Vorderseitenfläche in Kontakt mit dem Körper ist, und als Reaktion auf den erkannten Kontakt das akustische Signal zu verarbeiten, um eine Audioausgabe zu erzeugen und die Audioausgabe an den mindestens einen Lautsprecher zu übertragen, wobei

der Näherungssensor (56) mindestens einen Sender (58), der dazu eingerichtet ist, optische Strahlung, einschließlich Infrarotstrahlung, in Richtung des Körpers auszusenden, und einen Detektor (60), der dazu eingerichtet ist, die von dem Körper reflektierte optische Strahlung zu erfassen, umfasst,

**dadurch gekennzeichnet, dass** die Verarbeitungsschaltung ferner dazu eingerichtet ist, ein Niveau der Sauerstoffsättigung im Blut des Subjekts unter Verwendung der von dem Detektor erfassten reflektierten Strahlung zu messen.

2. Vorrichtung nach Anspruch 1, umfassend einem Bewegungssensor, der in dem Gehäuse (32) enthalten und dazu eingerichtet ist, als Reaktion auf eine Bewegung der Vorderseitenfläche ein Bewegungssignal auszugeben.

3. Vorrichtung nach Anspruch 2, wobei die Verarbeitungsschaltung (50) dazu eingerichtet ist, das Bewegungssignal zu verarbeiten, um einen Atmungszyklus des Subjekts zu erkennen.

4. Vorrichtung nach Anspruch 2, wobei die Vorderseitenfläche eine Membran (38) umfasst, die dazu eingerichtet ist, als Reaktion auf die vom Körper ausgesendeten akustischen Wellen zu vibrieren, und wobei der Bewegungssensor dazu eingerichtet ist, die Bewegung der Membran zu erfassen, und die Verarbeitungsschaltung dazu eingerichtet ist, das Bewegungssignal zu verarbeiten, um eine Infraschallkomponente der akustischen Wellen zu erkennen.

5. Vorrichtung nach Anspruch 1, wobei die Verarbeitungsschaltung (50) dazu eingerichtet ist, das akustische Signal zu verarbeiten, um ein frequenzgestrecktes Audio-Ausgangssignal zu erzeugen, bei dem Infraschall-Frequenzkomponenten des akustischen Signals auf hörbare Frequenzen verschoben werden, während die Periodizität der periodischen physiologischen Aktivität erhalten bleibt, und das frequenzgestreckte Audio-Ausgangssignal an den mindestens einen Lautsprecher zu übertragen.

6. Vorrichtung nach Anspruch 1, wobei die Verarbeitungsschaltung (50) dazu eingerichtet ist, eine oder mehrere Komponenten der Vorrichtung auszuschalten, wenn das Annäherungssignal anzeigt, dass die Vorderseitenfläche nicht in Kontakt mit dem Körper ist, und die eine oder mehreren Komponenten einzuschalten, wenn das Annäherungssignal anzeigt, dass ein Kontakt zwischen der Vorderseitenfläche und dem Körper hergestellt wurde.

7. Vorrichtung nach Anspruch 1, umfassend ein Paar Kopfhörer (30), die dazu eingerichtet sind, in die jeweiligen Ohren eines Bedieners der Vorrichtung eingeführt zu werden und die hörbaren Töne an die Ohren zu übertragen, wobei die Verarbeitungsschaltung dazu eingerichtet ist, zu erkennen, dass die Kopfhörer auseinandergespreizt wurden, und als Reaktion darauf eine oder mehrere Komponenten der Vorrichtung einzuschalten.

8. Vorrichtung nach Anspruch 1, wobei die Verarbeitungsschaltung (50) dazu eingerichtet ist, als Reaktion auf das Annäherungssignal eine Qualität des Kontakts zwischen der Vorderseitenfläche und dem Körper zu erkennen und eine Anzeige der erkannten Qualität an einen Bediener der Vorrichtung auszugeben.

9. Vorrichtung nach einem der Ansprüche 1 bis 8, wobei das Gehäuse eine Rückseitenfläche gegenüber der Vorderseitenfläche aufweist, und wobei die Vorrichtung einen berührungsempfindlichen Anzeigebildschirm (36) umfasst, der an der Rückseitenfläche des Gehäuses angebracht ist.

10. Vorrichtung nach Anspruch 9, wobei die Verarbeitungsschaltung dazu eingerichtet ist, eine Benutzerschnittstelle auf dem Anzeigebildschirm (36) zu präsentieren und über den Anzeigebildschirm (36) von einem Bediener der Vorrichtung eine Eingabe zu empfangen, die einen bei der Verarbeitung des akustischen Signals anzuwendenden Verarbeitungsmodus und eine Frequenzverschiebung angibt, wobei der Verarbeitungsmodus aus einer Gruppe von Verarbeitungsmodi ausgewählt ist, die aus einem Herzsignal-Verarbeitungsmodus und einem Atmungssignal-Verarbeitungsmodus besteht.

11. Vorrichtung nach Anspruch 9, wobei die Verarbeitungsschaltung dazu eingerichtet ist, als Reaktion auf das akustische Signal eine akustische Signatur zu berechnen und auf dem Bildschirm (36) darzustellen, die eine grafische Darstellung einer spektralen Verteilung einer Energie der akustischen Wellen über eine Periode physiologischer Aktivität umfasst.

12. Vorrichtung nach Anspruch 11, wobei die akustischen Wellen eine Infraschallkomponente umfassen und wobei die spektrale Verteilung die Energie bei Infraschallfrequenzen enthält.

13. Verfahren zum Sammeln, Verarbeiten und Analysieren von Signalen von einer Körperoberfläche, umfassend:

Empfangen, von einem Mikrofon (34) in einem Gehäuse (32) mit einer Vorderseitenfläche, die in Kontakt mit einem Körper eines lebenden Subjekts (24) gebracht wird, eines akustischen Signals als Reaktion auf von dem Körper ausgesendeten akustischen Wellen;

Empfangen eines Annäherungssignals, das einen Kontakt zwischen der Vorderseitenfläche des Gehäuses und dem Körper anzeigt;

Erkennen, als Reaktion auf das Annäherungssignal, dass die Vorderseitenfläche in Kontakt mit dem Körper ist; und

Verarbeiten, als Reaktion auf den erkannten Kontakt, des akustischen Signals, um eine Audioausgabe zu erzeugen und an mindestens einen Lautsprecher zu übertragen;

wobei das Empfangen des Annäherungssignals das Aussenden von optischer Strahlung in Richtung des Körpers und das Erfassen der von dem Körper reflektierten optischen Strahlung umfasst; und

wobei das Aussenden der optischen Strahlung das Aussenden von Infrarotstrahlung umfasst;

**dadurch gekennzeichnet, dass** das Verfahren das Messen eines Niveaus der Sauerstoffsättigung im Blut des Subjekts unter Verwendung der erfassten reflektierten Strahlung umfasst.

## Revendications

1. Appareil médical (20) comprenant :

un boîtier (32) ayant une surface avant conçue pour être portée au contact avec le corps d'un sujet vivant (24) ;

un microphone (34) contenu dans le boîtier et conçu pour détecter des ondes acoustiques émises par le corps et pour produire un signal acoustique en réponse à celles-ci ;

un capteur de proximité (56) conçu pour produire un signal de proximité indiquant un contact entre la surface avant et le corps ;

au moins un haut-parleur (49) conçu pour produire des sons audibles ; et

un circuit de traitement (50) couplé pour détecter, en réponse au signal de proximité, que la surface avant est en contact avec le corps, et en réponse à la détection du contact, pour traiter le signal acoustique de façon à générer une sortie audio et à transmettre la sortie audio à l'au moins un haut-parleur.

le capteur de proximité (56) comprenant au moins un émetteur (58) conçu pour émettre des radiations optiques, y compris des radiations infrarouges, vers le corps, et un détecteur (60) conçu pour capter les radiations optiques réfléchies par le corps,

**caractérisé en ce que** le circuit de traitement est également conçu pour mesurer un niveau de saturation d'oxygène dans le sang du sujet en utilisant les radiations réfléchies par le détecteur.

2. Appareil selon la revendication 1, et comprenant un capteur de mouvement contenu dans le boîtier (32) et conçu pour produire un signal de mouvement en réponse à un mouvement de la surface avant.

3. Appareil selon la revendication 2, dans lequel le circuit de traitement (50) est conçu pour traiter le signal de mouvement afin de détecter un cycle respiratoire du sujet.

4. Appareil selon la revendication 2, dans lequel la surface avant comprend une membrane (38) conçue pour vibrer en réponse aux ondes acoustiques émises par le corps, et dans lequel le capteur de mouvement est conçu pour capter le mouvement de la membrane, et le circuit de traitement est conçu pour traiter le signal de mouvement afin de détecter une composante ultrasonique des ondes acoustiques.

5. Appareil selon la revendication 1, dans lequel le circuit de traitement (50) est conçu pour traiter le signal acoustique de façon à générer une sortie audio allongée en fréquence, dans laquelle les composantes de fréquence ultrasonique du signal acoustique sont décalées vers des fréquences audibles tout en préservant la périodicité de l'activité physiologique périodique, et de façon à transmettre la sortie audio allongée en fréquence à l'au moins un haut-parleur.

6. Appareil selon la revendication 1, dans lequel le circuit de traitement (50) est conçu pour réduire la puissance d'au moins un composant de l'appareil quand le signal de proximité indique que la surface avant n'est pas en contact avec le corps, et pour augmenter la puissance de l'au moins un composant quand le signal de proximité indique qu'un contact a été établi entre la surface avant et le corps.

7. Appareil selon la revendication 1, et comprenant une paire d'écouteurs (30) conçus pour être insérés dans les oreilles respectives d'un opérateur de l'appareil et pour transmettre es sons audibles aux oreilles, le circuit de traitement étant conçu pour détecter que les écouteurs ont été séparés et pour augmenter la puissance d'au moins un composant de l'appareil en réponse à cette séparation.

8. Appareil selon la revendication 1, dans lequel le circuit de traitement (50) est conçu pour détecter, en réponse au signal de proximité, une qualité du contact entre la surface avant et le corps, et pour produire une indication de la qualité détectée à un opérateur de l'appareil.

9. Appareil selon l'une quelconque des revendications 1 à 8, dans lequel le boîtier a une surface arrière opposée à la surface avant, et dans lequel l'appareil comprend un écran d'affichage tactile (36) monté sur la surface arrière du boîtier.

10. Appareil selon la revendication 9, dans lequel le circuit de traitement est conçu pour présenter une interface d'utilisateur sur l'écran d'affichage (36) et pour recevoir une entrée d'un opérateur de l'appareil via l'écran d'affichage (36) indiquant un mode de traitement et un décalage de fréquence à appliquer dans le traitement du signal acoustique, le mode de traitement étant choisi dans un groupe de modes de traitement constitué d'un mode de traitement de signal cardiaque et d'un mode de traitement de signal respiratoire.

11. Appareil selon la revendication 9, dans lequel le circuit de traitement est conçu pour calculer, en réponse au signal acoustique, et pour rendre à l'écran d'affichage (36) une signature acoustique, comprenant une représentation graphique d'une distribution spectrale d'une énergie des ondes acoustiques sur une période d'activité physiologique.

12. Appareil selon la revendication 11, dans lequel les ondes acoustiques comprennent une composante infrasonique, et dans lequel la distribution spectrale inclut l'énergie par fréquences infrasoniques.

13. Procédé de collecte, de traitement et d'analyse de signaux d'une surface corporelle, comprenant de :

recevoir, depuis un microphone (34) dans un boîtier (32) ayant une surface avant qui est mise en contact avec un corps d'un sujet vivant (24), un signal acoustique en réponse à des ondes acoustiques émises par le corps ;
recevoir un signal de proximité indicatif de contact entre la surface avant du boîtier et le corps ;
détecter, en réponse au signal de proximité, que la surface avant est en contact avec le corps ; et
en réponse au signal détecté, traiter le signal acoustique de façon à générer et transmettre une sortie audio à au moins un haut-parleur ;
dans lequel recevoir le signal de proximité comprend d'émettre des radiations optiques vers le corps et détecter les radiations optiques réfléchies par le corps ; et
dans lequel émettre les radiations optiques comprend d'émettre des radiations infrarouges ;
**caractérisé en ce que** le procédé comprend de mesurer un niveau de saturation d'oxygène dans le sang du sujet en utilisant les radiations réfléchies et détectées.

FIG. 1

EP 3 678 551 B1

FIG. 2

FIG. 3

FIG. 4

FIG. 5

**REFERENCES CITED IN THE DESCRIPTION**

**Patent documents cited in the description**

- US 5853005 A
- US 6699204 B
- US 20050222515
- WO 2017141165 A
- US 2003002685 A
- US 2007058818 A
- US 953502